# EUROPEAN PATENT SPECIFICATION

(11) **EP 2 308 405 B1**
(45) Date of publication and mention of the grant of the patent: **21.03.2018**
(21) Application number: 09794450.8
(22) Date of filing: 07.07.2009
(51) Int. Cl.: A61B 18/14, A61B 18/12

(54) **HIGH-FREQUENCY TREATMENT INSTRUMENT**
HOCHFREQUENZBEHANDLUNGSINSTRUMENT
INSTRUMENT DE TRAITEMENT HAUTE FRÉQUENCE

(30) Priority: 08.07.2008 JP 2008177827
(43) Date of publication of application: 13.04.2011
(73) Proprietor: Olympus Corporation, Tokyo 192-8507 (JP)
(72) Inventor: SUZUKI, Keita, Tokyo 192-8507 (JP); KIMURA, Megumi, Tokyo 192-8507 (JP)
(74) Representative: Frenkel, Matthias Alexander
(86) International application number: PCT/JP2009/062393
(87) International publication number: WO 2010/005006

(56) References cited:
- JP-A- 5 123 325
- JP-A- 10 192 295
- JP-A- 58 083 950
- JP-A- 62 127 040
- JP-A- 2004 229 976
- JP-A- 2004 229 976
- JP-A- 2007 330 723
- JP-A- 2008 000 582
- JP-T- 8 509 623
- JP-T- 2002 528 166
- JP-U- 62 186 708
- JP-U- 62 186 708
- US-A1- 2005 187 547

## Description

### Field of the Invention

The present invention relates to a high-frequency treatment instrument that is used by being supplied with high frequency current.

### Background Art

Conventionally, a high-frequency treatment instrument is known in which high frequency current is supplied to a treatment portion from a high frequency power supply so that various procedures can be performed on tissue inside the body cavity of a patient.

An example of a typical high-frequency treatment instrument is the high frequency forceps described in Patent document 1. This high frequency forceps is provided with a pair of forceps components that serve as a treatment portion that is used to perform treatment inside a body cavity, a joining portion that is pivotably joined to a proximal end of each of the forceps components, and a wire that is connected to the joining portion so as to enable the forceps components to perform opening and closing operations. When a user moves the wire forwards or backwards in an axial direction, the joining portion and the forceps components pivot and the forceps components are opened or closed.

In the high frequency forceps described in Patent document 1, the forceps components, the joining portion, and the wire are all formed from metal which is a conductor. The wire is connected to a high frequency power supply in an operating section on the proximal end side which is operated by a user, and the supplied high frequency current flows to the forceps components via the wire and the joining portion.

Patent document 1: Japanese Patent Application, Publication No. 2000-93431
JP 62-186708 U relates to two local treatment electrodes 22, 23 formed in the distal end of two cups 17, 18, respectively. Isolated sections 20, 21 are arranged at the cups 17, 18.

US2005/0187547 A1 discloses a high-frequency treatment device having a pair of jaws with electrodes.

### Summary of the Invention

However, in the high frequency forceps described in Patent document 1, when the forceps components are open, because the distal end side of the joining portion which is connected to the proximal end of the forceps components protrudes outwards in the radial direction of the wire, it easily comes into contact with tissue inside the body cavity. As is described above, because high frequency current is flowing to the joining portion, if the joining portion comes into contact with tissue, current leaks from the contact portion and the efficiency of the treatment by the forceps components is reduced.

The present invention was conceived in view of the above described circumstances, and it is an object thereof to provide a high-frequency treatment instrument that prevents supplied high frequency current leaking from portions other than the treatment portion, and that is able to perform treatment efficiently.

### Means for Solving the Problem

The present invention is defined by the appended claims. The examples, embodiments, or aspects of the present description that do not fall within the scope of said claims are merely provided for illustrative purposes and do not form part of the invention.

A first aspect of the present invention is a high-frequency treatment instrument that is used by being supplied with high frequency current from a power supply, and that includes: a treatment portion main body that is used to treat biological tissue; a treatment electrode that is provided on a surface of the treatment portion main body that is in contact with the biological tissue, such that it is not electrically connected to the treatment portion main body; and a power supply device that electrically connects together the treatment electrode and the power supply such that a conductive external surface thereof is not exposed, and that is positioned such that it is not electrically connected to the treatment portion main body.

According to the above described high-frequency treatment instrument, high frequency current that is supplied from a power supply to a treatment electrode does not leak to the treatment portion main body, and any reduction in the efficiency of the treatment is prevented.

A second aspect of the present invention is a high-frequency treatment instrument that is used by being supplied with high frequency current from a power supply, and that includes: a treatment portion that is formed by a pair of forceps components having a conductive portion that is formed so as to include a conductor, and a non-conductive portion that is formed on a surface of the conductive portion; joining components that are pivotably joined to each of the forceps components without being electrically connected thereto; a wire whose distal end side is pivotably connected to a proximal end of the joining components without being electrically connected thereto, and whose proximal end side is electrically connected to the power supply; and an energizing component that electrically connects together the conductive portion and the wire, and is provided such that it is not electrically connected to the joining components, and that supplies the high frequency current to the conductive portion. In addition, the pair of forceps components has an electrode surface on at least one of the mutually facing surfaces at the distal end side thereof where the conductive portion is exposed.

According to the above described high-frequency treatment instrument, high frequency current that is supplied from the power supply to the conductive portion does not leak from portions other than the electrode surface, and any reduction in the efficiency of the treatment is prevented.

A third aspect of the present invention is a high-frequency treatment instrument that is used by being supplied with high frequency current from a power supply, and that includes: a treatment portion that is formed by a pair of forceps components having a conductive portion that is formed so as to include a conductor, and a non-conductive portion that is formed on a surface of the conductive portion; a wire whose distal end side is pivotably connected to a proximal end of the pair of forceps components; and an energizing component that electrically connects together the conductive portion and the power supply, and is provided such that it is not electrically connected to the wire, and that supplies the high frequency current to the conductive portion. In addition, the pair of forceps components has an electrode surface on at least one of the mutually facing surfaces at the distal end side thereof where the conductive portion is exposed.

According to the above described high-frequency treatment instrument, insulation property thereof is improved even further and any leakage of high frequency current is properly suppressed.

In the high-frequency treatment instrument of the present invention, it is also possible for there to be further provided joining components that are pivotably joined to each of the forceps components without being electrically connected to the conductive portions thereof. In this case, it is also possible for the wire to be connected to the pair of forceps components via the joining components that are pivotably joined to the distal end side of the wire, and for the energizing component to be positioned such that it is not electrically connected to the wire and the joining components.

It is also possible for the joining components to be joined to the wire via a connecting component that is attached to the distal end of the wire.

It is also possible for the energizing component to be electrically connected to the wire via the connecting component, and to be constructed so as to be able to move in an axial direction relatively to the connecting component.

In this case, because high frequency current can be supplied via the wire, forwards and backwards movements of the treatment portion can be made smoothly.

It is also possible for one end portion of the energizing component to be fixed to the connecting component and to be electrically connected to the wire via the connecting component, and for the energizing component to have sufficient flexibility to enable it to absorb movement in the axial direction of the connecting component which is brought about by an opening or closing operation of the forceps components.

In this case, it is possible to supply high frequency current more reliably to the electrode surface.

It is also possible for the connecting components to be provided as a pair so as to correspond individually to the pair of forceps components, and for the proximal ends of the joining components to be mutually offset when joined to the connecting components so that they are not coaxial. In this case, the rigidity of the treatment portion can be improved.

It is also possible for the joining components to be formed from a non-conductive material. In this case, the processing and work to make the treatment portion non-conductive can be performed easily.

### Effects of the Invention

According to the high-frequency treatment instrument of the present invention, it is possible to prevent supplied high frequency current leaking from portions other than a treatment portion, and treatment can be performed efficiently.

### Brief Description of the Drawings

FIG. 1 is an overall view of a high-frequency treatment instrument of a first embodiment of the present invention.
FIG. 2 is an enlarged view which showing a partial cross-section of a distal end side of this high-frequency treatment instrument.
FIG. 3 is a cross-sectional view taken along a line A-A in FIG. 2.
FIG. 4 is a drawing which showing a treatment portion of this high-frequency treatment instrument in an open state.
FIG. 5 is a drawing which showing a treatment portion of a high-frequency treatment instrument of a variant example of this embodiment in an open state.
FIG. 6 is an overall view of a high-frequency treatment instrument of a second embodiment of the present invention.
FIG. 7 is an enlarged view which showing a treatment portion of this high-frequency treatment instrument.
FIG. 8 is a cross-sectional view taken along a line B-B in FIG. 2.
FIG. 9 is a drawing which showing an operating section of a high-frequency treatment instrument of a third embodiment of the present invention.
FIG. 10 is an enlarged view which showing a treatment portion of this high-frequency treatment instrument.
FIG. 11 is a drawing which showing a connecting portion between an operating wire and forceps components in this treatment portion.
FIG. 12 is a drawing which showing how a conductive portion and a power supply wire are connected in this treatment portion.

### Best Embodiments for Carrying Out the Invention

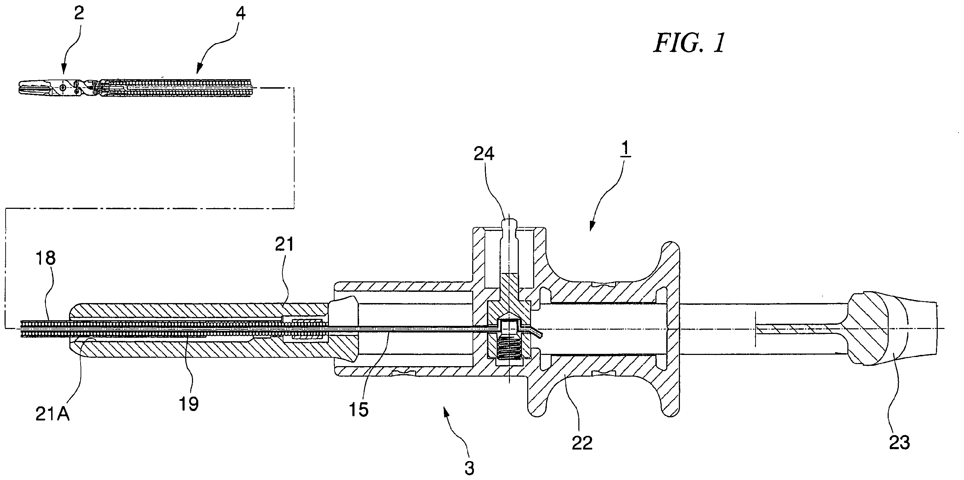

Hereinafter, a high-frequency treatment instrument according to a first embodiment of the present invention will be described with reference made to FIG. 1 through FIG. 5. A high-frequency treatment instrument 1 of the present embodiment is used by being supplied with high frequency current from a power supply (not shown). As is shown in FIG. 1, the high-frequency treatment instrument 1 is provided with a treatment portion 2 that is used to perform treatment on tissue inside a body cavity, an operating section 3 that is used to operate the treatment portion 2, and an insertion portion 4 that is used to connect together the treatment portion 2 and the operating section 3.

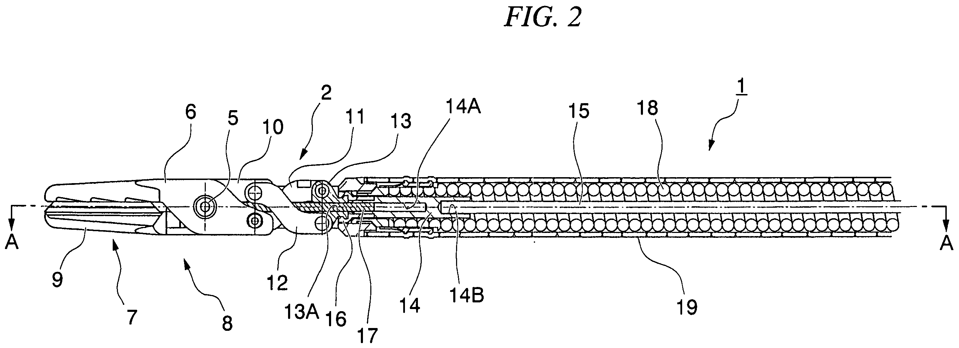

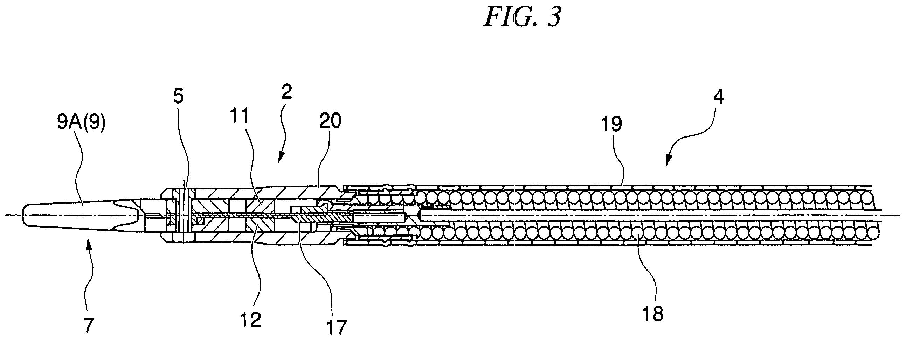

FIG 2 is an enlarged view showing a partial cross-section of the treatment portion 2, FIG. 3 is a cross-sectional view taken along line A-A in FIG. 2. As is shown in FIG. 2 and FIG. 3, the treatment portion 2 has a treatment portion main body 8 which is formed by a pair of forceps components, namely, a first forceps component 6 and a second forceps component 7 that are joined together such that they are able to pivot freely relative to each other around a pivot shaft 5.

The first forceps component 6 which is positioned on the upper side in FIG. 2 is non-conductive as a result of being formed from a ceramic component such as alumina or zirconia, or from a resin such as polytetrafluoroethylene (PTFE) or PEEK (registered trademark) or the like. Note that, instead of this type of structure, it is also possible to form the first forceps component 6 from a metal such as stainless steel or the like and to then provide a non-conductive coating on the surface thereof.

In contrast, the second forceps component 7 which is positioned on the bottom side is formed by a conductive portion 9 that is provided on the distal end side, and by a non-conductive portion 10 that is provided on the proximal end side of the conductive portion 9. The conductive portion 9 is formed by a conductor such as stainless steel or the like, and performs treatment on tissue inside a body cavity using high frequency current which is supplied from an electrode. The method of supplying power to the conductive portion 9 is described below.

The non-conductive portion 10 is formed from the same material as the first forceps component 6. Namely, the conductive portion 9 which is part of the treatment portion main body 8 functions as a treatment electrode that is not electrically connected to the first forceps component 6 and the nonconductive portion 10. In addition, the surface of the conductive portion 9 that faces the first forceps component 6 is exposed as an electrode surface 9A that is in contact with biological tissue inside the body cavity during treatment (described below).

Distal ends of a first joining component 11 and a second joining component 12 are pivotably joined respectively to proximal ends of the first forceps component 6 and second forceps component 7. The first joining component 11 and the second joining component 12 are formed from a non-conductive material in the same way as the first forceps component 6 and, as is shown in FIG. 2, the proximal ends of each of these joining components are pivotably joined to a connecting component 13 by intersecting each other substantially in an X shape such that these proximal ends do not overlap each other and are not coaxial.

An operating wire (i.e., a wire) 15 is connected via a wire connecting component 14 to the treatment portion 2. The specific method used for this connection is described below.

A first recessed portion 14A and a second recessed portion 14B are formed respectively in a distal end and in a proximal end of the wire connecting component 14 such that they both extend in the longitudinal direction thereof. A distal end of the operating wire 15 is inserted into the second recessed portion 14B and is fixed therein. A proximal end of the connecting component 13 is connected to the distal end of the wire connecting component 14 via a fixing component 16. A through hole 13A is formed in the connecting component 13 so as to so as to extend in an axial direction thereof, and the connecting component 13 is attached to the wire connecting component 14 such that the through hole 13A and the first recessed portion 14A are connected together substantially coaxially.

An energizing component 17 that is used to supply high frequency current to the conductive portion 9 is slidably attached to the through hole 13A and the first recessed portion 14A. The energizing component 17 is a conductive, substantially bar-shaped component whose distal end side is formed more thinly. As is shown in FIG. 3, the energizing component 17 extends between the respective joining components 11 and 12 as far as the vicinity of the pivot shaft 5. In addition, it is electrically connected to the proximal end side of the conductive portion 9 which also extends as far as the vicinity of the pivot shaft 5.

The insertion portion 4 is formed by a coil sheath 18, and by a non-conductive tube 19 which covers an outer circumference of the coil sheath 18. The operating wire 15 is inserted through the insertion portion 4. As is shown in FIG. 3, a distal end of the insertion portion 4 is integrally linked to the pivot shaft 5 via a cover 20, and the pivot shaft 5 is unable to move relatively to the insertion portion 4. Note that, in FIG. 2, the cover 20 has been omitted in order to make the structure easier to view.

Returning to FIG. 1, the operating section 3 is provided with a narrow, elongated operating section main body 21, and with a slider 22 that is mounted such that it is able to move relatively in an axial direction relative to the operating section main body 21. provided in the operating section main body 21, and the coil sheath 18 and non-conductive tube 19 are inserted through this insertion portion hole 21A and proximal ends of each of these are connected to the operating section main body 21. A proximal end of the operating wire 15 which is inserted into the insertion portion 4 is connected to the slider 22. A finger grip handle 23 is provided at a proximal end of the operating section main body 21.

A plug 24 to which is connected a power supply cable (not shown) that is connected to a high frequency power supply (not shown) is attached to the slider 22, and this plug 24 is electrically connected to the operating wire 15. Accordingly, when the high frequency power supply is connected via the power supply cable to the plug 24 and is supplying power thereto, high frequency current is supplied to the conductive portion 9 through the operating wire 15, the wire connecting component 14, and the energizing component 17.

Operations performed when the high-frequency treatment instrument 1 having the above described structure is put to use will now be described.

Firstly, an endoscope (not shown) is inserted into the body cavity of a patient who is in contact with a known counter electrode plate (not shown), and the distal end of the endoscope is moved forward to the vicinity of the tissue inside the body cavity which is to be the subject of the treatment.

Next, the slider 22 is moved backwards relative to the operating section main body 21 with the pair of forceps components 6 and 7 being left in a closed state, and the treatment portion 2 and the insertion portion 4 are inserted into a forceps channel (not shown). Next, after the treatment portion 2 has been made to protrude from the forceps channel, the high frequency power supply and the plug 24 are connected together by means of the power supply cable.

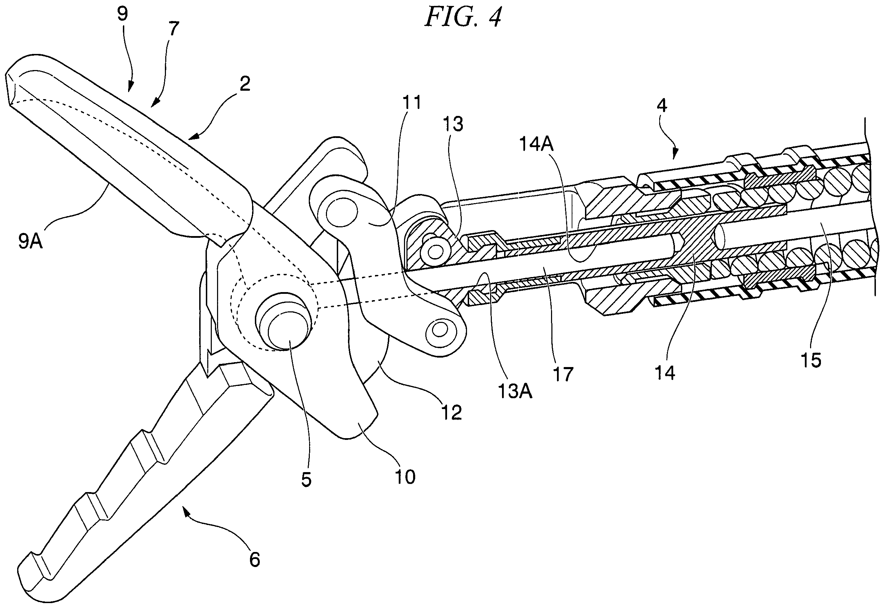

When treatment is to be performed, the slider 22 is made to move forwards relative to the operating section main body 21. As a result, the operating wire 15 which is connected to the slider 22 moves forward relative to the coil sheath 18. As is described above, because the pivot shaft 5 is unable to move relative to the insertion portion 4, the first forceps component 6 and the second forceps component 7 each pivot around the pivot shaft 5 and, as is shown in FIG. 4, the treatment portion 2 opens up.

At this time, as a result of the operating wire 15 moving forward, the wire connecting component 14 and the connecting component 13 which are integrally fixed to the operating wire 15 also move forward, and the relative distance between these components and the pivot shaft 5 becomes shortened. In contrast to this, because the energizing component 17 is able to slide inside the through hole 13A and the first recessed portion 14A, when the treatment portion 2 is opened, as is shown in FIG. 4, the energizing component 17 moves relatively towards the proximal end side such that it penetrates even more deeply inside the first recessed portion 14 A. As a result of this, the treatment portion 2 opens smoothly without any interference from the energizing component 17 and the operating wire 15.

Moreover, in conjunction with the opening of the treatment portion 2, the areas adjacent to the proximal ends of the respective forceps components 6 and 7 that are located on the proximal end side of the pivot shaft 5, and also the respective joining components 11 and 12 protrude outwards such that they move away from the axial line of the insertion portion 4.

However, the area adjacent to the proximal end of the second forceps component 7 is formed by the non-conductive portion 10, and the respective joining components 11 and 12 are also formed from a non-conductive material. Accordingly, even if these portions do make contact with tissue inside a body cavity which does not need to be treated while the conductive portion 9 is being energized, there is no leakage of high frequency current from the contacted portions.

When a user positions the subject tissue between the open forceps components 6 and 7 of the treatment portion 2, and pulls the slider 22 backwards towards the proximal end side of the operating section main body 21, the distal end side of the pair of forceps components 6 and 7 are once again closed and the subject tissue is gripped by the treatment portion 2. In addition, the electrode surface 9A is placed in contact with the body tissue which is to be treated.

In this state, when the user supplies high frequency current from the high frequency current supply, the high frequency current is supplied to the conductive portion 9 via the above described path, and the subject tissue is cauterized by the high frequency current on the electrode surface 9A.

After treatment has ended, the user extracts the high-frequency treatment instrument 1 from the forceps channel, and ends the operation by extracting the endoscope to the outside of the patient.

According to the high-frequency treatment instrument 1 of the present embodiment, when the treatment portion 2 is open, the area on the proximal end side of the respective forceps components 5 and 6 which may easily come into contact with tissue that has no connection to any treatment, and also their respective joining components 11 and 12 are held in an electrically unconnected state to the operating wire 15, the energizing component 17, and the conductive portion 9 and the like to which the high frequency current is supplied.

Accordingly, even if these portions do come into contact with tissue there is no leakage of high frequency current, and current is concentrated efficiently in the conductive portion 9. Because of this, it is possible to improve the efficiency of the treatment.

Moreover, because the proximal ends of the joining components 11 and 12 are joined to the connecting component 13 such that they are not mutually coaxial, in the join portions of these components, only two components, namely, the connecting component 13 and the first joining component 11, or the connecting component 13 and the second joining component 12 are mutually superimposed, and there is no portion where three of these components are mutually superimposed. Accordingly, it is possible to increase the thickness of these components without increasing the dimensions in the width direction of the treatment portion 2 (i.e., the width direction of the electrode surface 9A), so that the rigidity of the treatment portion 2 can be increased.

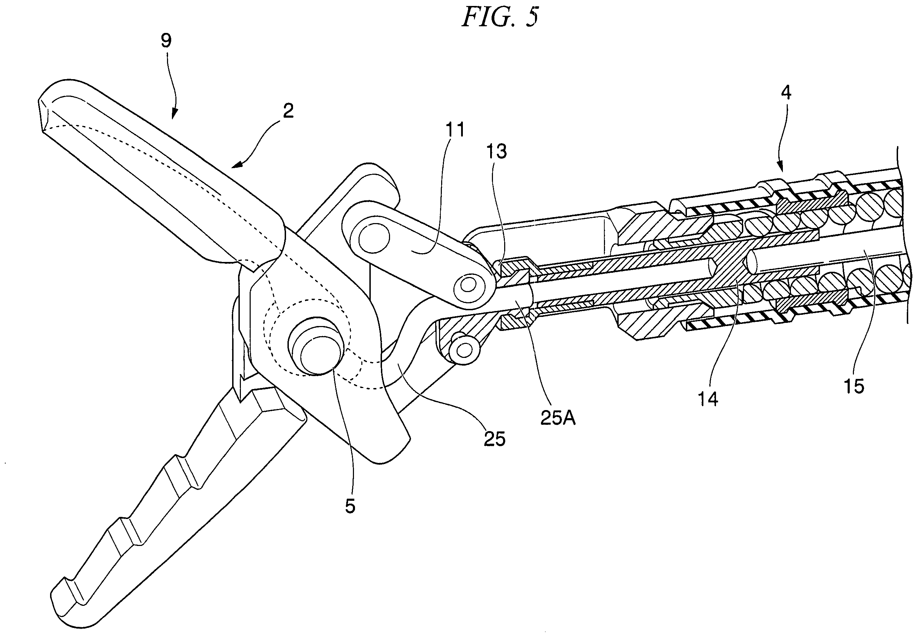

In the present embodiment, an example has been described in which the energizing component 17 is slidably positioned inside the first recessed portion 14A, and the outer surface of the energizing component 17 makes contact with the inner surface of the first recessed portion 14A so that these two components are electrically connected together, however, instead of this, as in the variant example shown in FIG. 5, it is also possible to form an energizing component 25 using wire or the like so that it has flexibility, and to fix a proximal end 25A thereof to the distal end of the wire connecting component 14 such that the energizing component 25 is not able to slide.

In this case, when the slider 22 is moved backwards so that the treatment portion 2 is closed, the operating wire 15 and the wire connecting component 14 move backwards so that the energizing component 25 becomes rectilinear. In contrast, when the operating wire 15 is moved forwards so that the treatment portion 2 is opened, the relative approach of the operating wire 15 towards the pivot shaft 5 is absorbed by the bending of the conductive component 25 so that any interference between the conductive component 25 and the operating wire 15 and wire connecting component 14 is prevented.

In the above-described variant example, because the energizing component 25 is fixed to the wire connecting component 14, the electrical connection between these two components is more reliable and high frequency current can be supplied more reliably to the conductive portion 9.

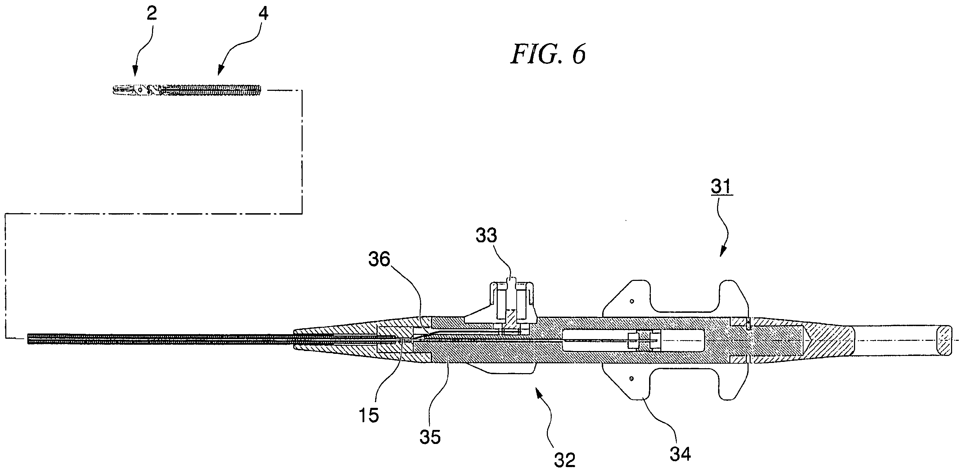

Next, a second embodiment of the present invention will be described with reference made to FIG. 6 through FIG. 8. A high-frequency treatment instrument 31 of the present embodiment differs from the above described high-frequency treatment instrument 1 in that a component that is used to supply power to the conductive portion is provided separately from the operating wire.

Note that component elements that are the same as those in the above described high-frequency treatment instrument are given the same symbols and any repeated description thereof is omitted.

FIG. 6 is an overall view of the high-frequency treatment instrument 31. In an operating section 32, a plug 33 that is connected to a power supply is provided at a distance from a slider 34, and is directly attached to an operation section main body 35. Accordingly, the plug 33 is not able to move relatively to the operating section main body 35.

A power supply wire 36 that is used to supply high frequency current to the conductive portion 9 is connected to the plug 33, and extends alongside the operating wire 15 inside the operating section main body 35 and the insertion portion 4 as far as the treatment portion 2. At least the portion of the power supply wire 36 that runs alongside the operating wire 15 is coated with insulation such as a non-conductive tube or a non-conductive coating so as to be in an electrically unconnected state relative to the operating wire 15.

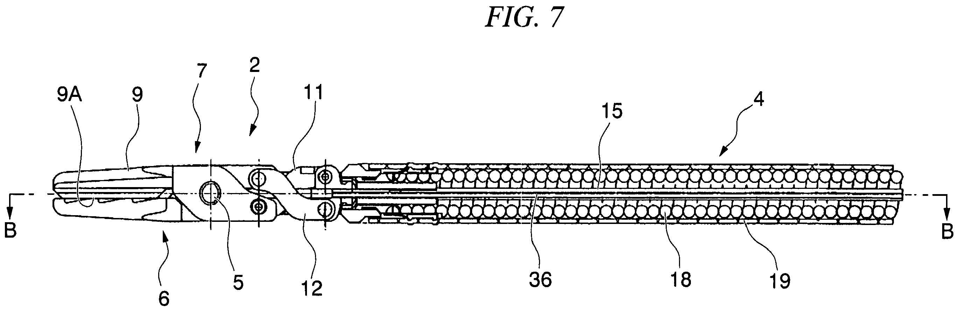

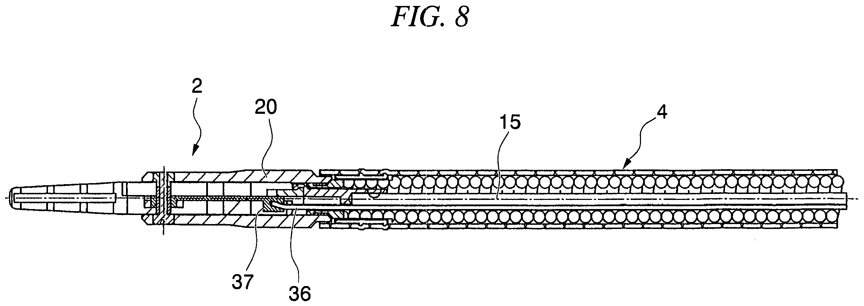

FIG. 7 is an enlarged view which showing a partial cross-section of the treatment portion 2, while FIG. 8 is a cross-sectional view taken along a line B-B in FIG. 7. As is shown in FIG. 7 and FIG. 8, a distal end of the power supply wire 35 which is inserted into the insertion portion 4 is electrically connected to the energizing component 37. Accordingly, high frequency current which is supplied from a power supply is supplied to the conductive portion 9 via the plug 53, the power supply wire 36, and the energizing component 37.

In the high-frequency treatment instrument 31 of the present embodiment as well, it is possible to obtain a similar effect as that obtained from the above described high-frequency treatment instrument 1.

Moreover, because the power supply wire 36 which is provided separately from the operating wire 15 is electrically connected to the conductive portion 9, the energizing of the conductive portion 9 and the electrode surface 9A can be performed more reliably.

Furthermore, the plug 33 to which the power supply wire 36 is connected is separated from the slider 34 and is directly attached to the operating section main body 35. As a result of this, because the plug 33 and the power supply wire 36 do not slide in conjunction with an opening or closing operation of the treatment portion 2, no unnecessary force is applied to the power supply wire 36 during an operation, so that it is difficult for breakages or the like to occur. Accordingly, it is possible to supply power more stably to the conductive portion 9.

Moreover, because there is no elongation or contraction or flexure of the power supply wire 36, it is possible for the power supply wire 36 to be formed having a narrow diameter of approximately, for example, 0.1 through 0.3 mm. By employing this type of narrow diameter, the sliding of the operating wire 15 inside the insertion portion 4 is not obstructed and it is possible to maintain a superior operability.

Note that if the above described advantages are disregarded, in the same way as in the high-frequency treatment instrument 1, the plug 33 can be attached to the slider 34.

In the present embodiment, an example has been described in which the conductive portion 9 and the power supply wire 36 are electrically connected together via the energizing component 37, and the energizing portion is formed by the power supply wire 36 and the energizing component 37. Instead of this, it is also possible for the power supply wire 36 to extend as far as the vicinity of the pivot shaft 5, and to be directly connected electrically with the conductive portion 9. In this case, the energizing portion is formed solely by the power supply wire 36.

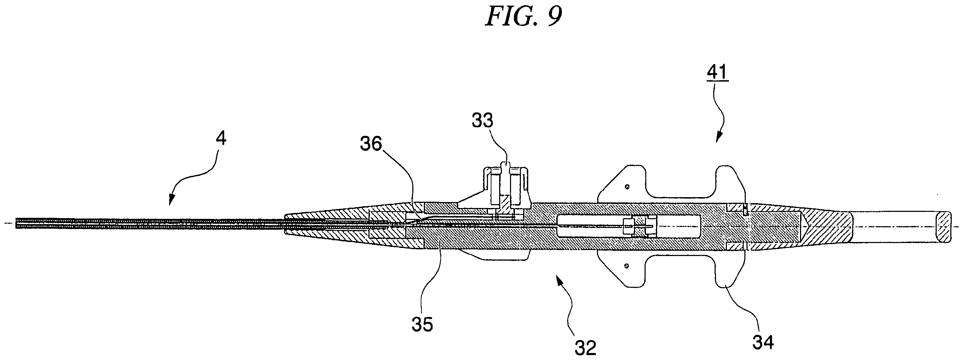

Next, a third embodiment of the present invention will be described with reference made to FIG. 9 through FIG. 12. A high-frequency treatment instrument 41 of the present embodiment differs from the high-frequency treatment instruments of each of the above described embodiments in the method used to connect together the operating wire and the treatment portion.

Note that component elements that are the same as those in the high-frequency treatment instruments of each of the above described embodiments are given the same symbols and any repeated description thereof is omitted.

FIG. 9 shows an operating section 32 of the high-frequency treatment instrument 41. The operating section 32 is the same as in the high-frequency treatment instrument 31 of the second embodiment, and the plug 33 is fixed to the operating section main body 35. In addition, the power supply wire 36 extends through the insertion portion 4 as far as a treatment portion 42 (described below).

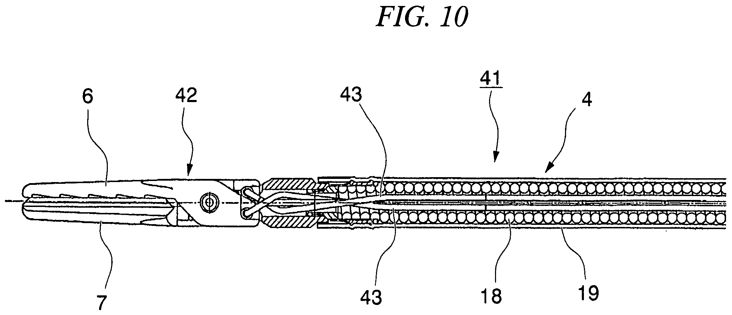

FIG. 10 is an enlarged view of the treatment portion 42. The treatment portion 42 of the present embodiment is basically formed solely by the first forceps component 6 and the second forceps component 7, and has a structure in which joining components and connecting components are not provided. Moreover, two operating wires 43 are provided to correspond to the respective forceps components, and these are connected to the proximal end sides of the respective forceps components 6 and 7. Those of the operating wires 43 extend through the insertion portion 4 as far as the operating section 32, and are connected to the slider 34.

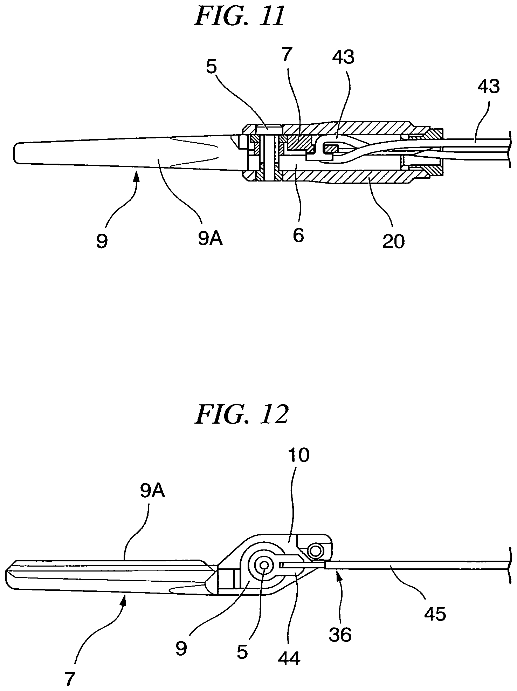

As is shown in FIG. 11, distal ends of the operating wires 43 are connected by caulking to the respective forceps components 6 and 7. By doing this, connections between these members can be made more reliable, however, if they are to be pivotably connected together, then it is also possible for them to be connected using another method.

FIG. 12 shows a method used to connect together the second forceps component 7 and the power supply wire 36. The power supply wire 36 is connected to the pivot shaft 5 via a pivot component 44 that is anchored to the pivot shaft 5. In addition, the power supply wire 36 is electrically connected to the proximal end side of the conductive portion 9 which extends as far as the vicinity of the pivot shaft 5. A non-conductive coating 45 of the power supply wire 36 is preferably provided as far as a point immediately in front of the connection portion between a pivot component 44 and the power supply wire 36 if this is necessary, so that the operating wires 43 which are connected to the proximal end of the second forceps component 7 and the power supply wire 36 can be kept in an electrically unconnected state.

In the high-frequency treatment instrument 41 of the present embodiment, when a user moves the slider 34 forward, the operating wires 43 are pushed forwards and the pair of forceps components 6 and 7 are pivoted around the pivot shaft 5 so that the treatment portion 42 is opened. At this time, a portion of the operating wires 43 that are connected to the vicinity of the proximal ends of the respective forceps components 6 and 7 protrude outwards such that they move away from the axial line of the insertion portion 4, and come into contact easily with tissue inside a body cavity.

However, because the power supply wire 36 that supplies high frequency current to the conductive portion 9 and the operating wires 43 are electrically unconnected, even if the operating wires 43 do come into contact with tissue inside a body cavity, there is no leakage of high frequency current.

In this manner, according to the high-frequency treatment instrument 41 of the present embodiment, the same effects can be obtained as those from the above described high-frequency treatment instrument 1.

Moreover, because there is no need to provide mechanisms such as joining components and connecting components in the treatment portion 42, the structure of the connection portion between the treatment portion and the operating wire is simplified and assembling these is made easier.

Embodiments of the present invention have been described above, however, the range of the technology of the present invention is not limited to the above described embodiments and various modifications and the like may be made thereto insofar as they do not depart from the scope of the present invention.

For example, in each of the above described embodiments an example has been described of what is known as a monopolar type of high-frequency treatment instrument in which a treatment electrode is provided in only the second forceps component out of a pair of forceps components, however, instead of this, it is also possible to construct the high-frequency treatment instrument of the present invention as what is known as a bipolar type of high-frequency treatment instrument in which treatment electrodes are provided in both of the pair of forceps electrodes. The operating method in this case is largely the same as for a normal bipolar high-frequency treatment instrument.

Moreover, in each of the above described embodiments an example has been described in which non-conductive portions are formed using non-conductive components, however, instead of this, it is also possible to provide a non-conductive portion by employing a method in which the entire second forceps component is formed from a conductive body, and a non-conductive coating is applied to the outer surface on the proximal end side thereof.

### Industrial Applicability

According to the high-frequency treatment instrument of the present invention, it is possible to prevent supplied high frequency current leaking from portions other than the treatment portion, and to perform treatment efficiently.

### Description of the Reference Numerals

1, 31, 41 High frequency treatment tool, 2, 42 Treatment portion, 6 First forceps component, 7 Second forceps component, 8 Treatment portion main body, 9 Conductive portion, 9A Electrode surface, 10 Non-conductive portion, 11 First joining component, 12 Second joining component, 13 Connecting component, 15, 43 Operating wire (wire), 17, 25 Energizing component, 36 Power supply wire.

## Claims

1. A high-frequency treatment instrument for supplying a high frequency current from a power supply, comprising:
a treatment portion main body(8) for treating biological tissue;
a treatment electrode (9) that is provided on a surface of the treatment portion main body that is in contact with the biological tissue; and
a power supply device (14, 15, 17, 24), wherein
the treatment portion main body (8) includes a first forceps component (6) of which an external surface is formed from a non-conductive material and a second forceps component (7) which includes a conductive portion (9) and a non-conductive portion (10),
the first forceps component (6) and the second forceps component (7) are joined together by a pivot shaft (5),
an electrode surface (9A) of the conductive portion (9) is in contact with the biological tissue by exposing a surface of the conductive portion (9) that faces the first forceps component (6), and
the power supply device electrically connects together the conductive portion (9) and the power supply such that a conductive external surface thereof is not exposed, **characterized in that**
the high-frequency treatment instrument further includes:
a wire (43) whose distal end side is pivotably connected to a proximal end of the pair of the first forceps component (6) and the second forceps component (7); and
an energizing component (37) that electrically connects together the conductive portion (9) and the power supply, and is provided such that it is not electrically connected to the wire (43), and that supplies the high frequency current to the conductive portion (9).

2. The high-frequency treatment instrument according to claim 1, comprising:
joining components (11, 12) that are pivotably joined to each of the first and second forceps component without being electrically connected thereto;
a wire (15, 36) whose distal end side is pivotably connected to a proximal end of the joining components without being electrically connected thereto, and whose proximal end side is electrically connected to the power supply; and
an energizing component (17, 37) that electrically connects together the conductive portion and the wire, and is provided such that it is not electrically connected to the joining components, and the supplies the high frequency current to the conductive portion.

3. The high-frequency treatment instrument according to claim 2, wherein the joining components are not electrically connected to the conductive portion (9).

4. The high-frequency treatment instrument according to claim 2, wherein the joining components are joined to the wire via a connecting component (13) that is attached to the distal end of the wire.

5. The high-frequency treatment instrument according to claim 4, wherein
the energizing component is electrically connected to the wire via the connecting component, and is constructed so as to be able to move in an axial direction relatively to the connecting component.

6. The high-frequency treatment instrument according to claim 4, wherein
one end portion of the energizing component is fixed to the connecting component and is electrically connected to the wire via the connecting component, and
the energizing component has sufficient flexibility to enable it to absorb movement in the axial direction of the connecting component which is brought about by an opening or closing operation of the forceps components.

7. The high-frequency treatment instrument according to any one of claims 4 through 6, wherein the connecting components are provided as a pair so as to correspond individually to the pair of forceps components, and
the proximal ends of the joining components are mutually offset when joined to the connecting components so that they are not coaxial.

8. The high-frequency treatment instrument according to claim 4, wherein the connecting components are provided as a pair so as to correspond individually to the pair of forceps components, and are joined to the wire via a connecting component that is attached to the distal end of the wire, and
the proximal ends of the joining components are mutually offset when joined to the connecting components so that they are not coaxial.

9. The high-frequency treatment instrument according to claim 2 or 3, wherein the joining components are formed from a non-conductive material.

## Patentansprüche

1. Hochfrequenzbehandlungsinstrument zum Liefern eines Hochfrequenzstroms von einer Stromversorgung, umfassend:
einen Behandlungsabschnittshauptkörper (8) zum Behandeln von biologischem Gewebe;
eine Behandlungselektrode (9), die an einer Oberfläche des Behandlungsabschnittshauptkörpers, der mit dem biologischen Gewebe in Kontakt ist, versehen ist; und
eine Stromversorgungseinrichtung (14, 15, 17, 24), wobei
der Behandlungsabschnittshauptkörper (8) eine erste Zangenkomponente (6), deren eine Außenfläche aus einem nichtleitenden Material gebildet ist, und eine zweite Zangenkomponente (7), die einen leitenden Abschnitt (9) und einen nichtleitenden Abschnitt (10) umfasst, umfasst,
die erste Zangenkomponente (6) und die zweite Zangenkomponente (7) durch eine Drehachse (5) miteinander verbunden sind,
eine Elektrodenoberfläche (9A) des leitenden Abschnitts (9) durch Freilegen einer Oberfläche des leitenden Abschnitts (9), der der ersten Zangenkomponente (6) zugewandt ist, mit dem biologischen Gewebe in Kontakt ist, und
die Stromversorgungseinrichtung den leitenden Abschnitt (9) und die Stromversorgung derart elektrisch miteinander verbindet, dass eine leitende Außenfläche davon nicht freiliegt, **dadurch gekennzeichnet, dass**
das Hochfrequenzbehandlungsinstrument ferner umfasst:
einen Draht (43), dessen Distalendseite drehbar mit einem proximalen Ende des Paares aus der ersten Zangenkomponente (6) und der zweiten Zangenkomponente (7) verbunden ist; und
eine Erregerkomponente (37), die den leitenden Abschnitt (9) und die Stromversorgung elektrisch miteinander verbindet, und derart vorgesehen ist, dass sie nicht elektrisch mit dem Draht (43) verbunden ist, und die den Hochfrequenzstrom an den leitenden Abschnitt (9) liefert.

2. Hochfrequenzbehandlungsinstrument nach Anspruch 1, umfassend:
Verbindungskomponenten (11, 12), die drehbar mit jeder der ersten und der zweiten Zangenkomponente verbunden sind, ohne elektrisch damit verbunden zu sein;
einen Draht (15, 36), dessen Distalendseite drehbar mit einem proximalen Ende der Verbindungskomponenten verbunden ist, ohne elektrisch damit verbunden zu sein, und dessen Proximalendseite elektrisch mit der Stromversorgung verbunden ist; und
eine Erregerkomponente (17, 37), die den leitenden Abschnitt und den Draht elektrisch miteinander verbindet, und derart vorgesehen ist, dass sie nicht elektrisch mit den Verbindungskomponenten verbunden ist, und die den Hochfrequenzstrom an den leitenden Abschnitt liefert.

3. Hochfrequenzbehandlungsinstrument nach Anspruch 2, wobei die Verbindungskomponenten nicht elektrisch mit dem leitenden Abschnitt (9) verbunden sind.

4. Hochfrequenzbehandlungsinstrument nach Anspruch 2, wobei die Verbindungskomponenten über eine Verbindungskomponente (13), die an dem distalen Ende des Drahts angebracht ist, mit dem Draht verbunden ist.

5. Hochfrequenzbehandlungsinstrument nach Anspruch 4, wobei die Erregerkomponente über die Verbindungskomponente elektrisch mit dem Draht verbunden ist, und derart konstruiert ist, dass sie sich in einer Achsrichtung relativ zu der Verbindungskomponente bewegen kann.

6. Hochfrequenzbehandlungsinstrument nach Anspruch 4, wobei ein Endabschnitt der Erregerkomponente an der Verbindungskomponente fixiert ist und über die Verbindungskomponente elektrisch mit dem Draht verbunden ist, und
die Erregerkomponente ausreichend Flexibilität aufweist, um das Absorbieren einer Bewegung in der Achsrichtung der Verbindungskomponente, die durch einen Öffnungs- oder Schließvorgang der Zangenkomponenten verursacht wird, zu ermöglichen.

7. Hochfrequenzbehandlungsinstrument nach einem der Ansprüche 4 bis 6, wobei die Verbindungskomponenten als ein Paar vorgesehen sind, um individuell dem Paar Zangenkomponenten zu entsprechen, und
die proximalen Enden der Verbindungskomponenten gemeinsam verschoben werden, wenn sie derart mit den Verbindungskomponenten verbunden werden, dass sie nicht koaxial sind.

8. Hochfrequenzbehandlungsinstrument nach Anspruch 4, wobei die Verbindungskomponenten als Paar vorgesehen sind, um individuell dem Paar Zangenkomponenten zu entsprechen, und über eine Verbindungskomponente, die an dem distalen Ende des Drahts angebracht ist, mit dem Draht verbunden sind, und
die proximalen Enden der Verbindungskomponenten gemeinsam verschoben werden, wenn sie derart mit den Verbindungskomponenten verbunden werden, dass sie nicht koaxial sind.

9. Hochfrequenzbehandlungsinstrument nach Anspruch 2 oder 3, wobei die Verbindungskomponenten aus einem nichtleitenden Material gebildet sind.

## Revendications

1. Instrument de traitement haute fréquence permettant de fournir un courant de haute fréquence provenant d'une alimentation électrique, comprenant :
un corps principal d'une partie de traitement (8) permettant de traiter un tissu biologique ;
une électrode de traitement (9) qui est fournie sur une surface du corps principal de la partie de traitement qui est en contact avec le tissu biologique ; et
un dispositif d'alimentation électrique (14, 15, 17, 24) dans lequel
le corps principal de la partie de traitement (8) comprend un premier composant de forceps (6) dont une surface externe est formée à partir d'un matériau non conducteur et un second composant de forceps (7) qui comprend une partie conductrice (9) et une partie non conductrice (10),
le premier composant de forceps (6) et le second composant de forceps (7) sont reliés l'un à l'autre par un axe de pivot (5),
une surface d'électrode (9A) de la partie conductrice (9) est en contact avec le tissu biologique en exposant une surface de la partie conductrice (9) qui fait face au premier composant de forceps (6), et
le dispositif d'alimentation électrique connecte électriquement la partie conductrice (9) et l'alimentation électrique de sorte qu'une surface externe conductrice de celle-ci n'est pas exposée, **caractérisé en ce que**
l'instrument de traitement haute fréquence comprend en outre :
un fil (43) dont le côté d'extrémité distale est connecté de manière pivotante à une extrémité proximale de la paire du premier composant de forceps (6) et du second composant de forceps (7) ; et
un composant de mise sous tension (37) qui connecte électriquement la partie conductrice (9) et l'alimentation électrique, et est fourni de sorte qu'il n'est pas connecté électriquement au fil (43), et qui fournit le courant de haute fréquence à la partie conductrice (9).

2. Instrument de traitement haute fréquence selon la revendication 1, comprenant :
des composants de liaison (11, 12) qui sont reliés de manière pivotante à chacun du premier et du second composant de forceps sans être connectés électriquement à ceux-ci ;
un fil (15, 36) dont le côté d'extrémité distale est connecté de manière pivotante à une extrémité proximale des composants de liaison sans être connecté électriquement à celle-ci, et dont le côté d'extrémité proximale est connecté électriquement à l'alimentation électrique ; et
un composant de mise sous tension (17, 37) qui relie électriquement la partie conductrice et le fil, et qui est fourni de sorte qu'il n'est pas connecté électriquement aux composants de liaison, et qui fournit le courant de haute fréquence à la partie conductrice.

3. Instrument de traitement haute fréquence selon la revendication 2, dans lequel les composants de liaison ne sont pas connectés électriquement à la partie conductrice (9).

4. Instrument de traitement haute fréquence selon la revendication 2, dans lequel les composants de liaison sont reliés au fil par le biais d'un composant de connexion (13) qui est fixé à l'extrémité distale du fil.

5. Instrument de traitement haute fréquence selon la revendication 4, dans lequel
le composant de mise sous tension est connecté électriquement au fil par le biais du composant de connexion, et est élaboré de façon à pouvoir se déplacer dans une direction axiale par rapport au composant de connexion.

6. Instrument de traitement haute fréquence selon la revendication 4, dans lequel
une partie d'extrémité du composant de mise sous tension est fixée au composant de connexion et est connectée électriquement au fil par le biais du composant de connexion, et
le composant de mise sous tension possède une flexibilité suffisante pour lui permettre d'absorber un mouvement dans la direction axiale du composant de connexion qui est provoqué par une opération d'ouverture ou de fermeture des composants de forceps.

7. Instrument de traitement haute fréquence selon l'une quelconque des revendications 4 à 6, dans lequel les composants de connexion sont fournis sous forme de paire de façon à correspondre individuellement à la paire des composants de forceps, et
les extrémités proximales des composants de jonction sont décalées mutuellement lorsqu'elles sont reliées aux composants de connexion afin de ne pas être coaxiales.

8. Instrument de traitement haute fréquence selon la revendication 4, dans lequel les composants de connexion sont fournis sous forme de paire de façon à correspondre individuellement à la paire de composants de forceps, et sont reliés au fil par le biais d'un composant de connexion qui est fixé à l'extrémité distale du fil, et
les extrémités proximales des composants de liaison sont décalées mutuellement lorsqu'elles sont reliées aux composants de connexion afin de ne pas être coaxiales.

9. Instrument de traitement haute fréquence selon la revendication 2 ou 3, dans lequel les composants de liaison sont formés à partir d'un matériau non conducteur.
